# EUROPÄISCHE PATENTANMELDUNG

(11) **EP 3 964 760 A1**
(43) Veröffentlichungstag der Anmeldung: **09.03.2022**
(21) Anmeldenummer: 21194288.3
(22) Anmeldetag: 01.09.2021
(51) Int. Cl.: F24F 8/40, F24F 8/22

(54) **LUFTBEHANDLUNGSSYSTEM ZUM REINIGEN VON RAUMLUFT**

(30) Priorität: 08.09.2020 DE 102020123367
(71) Anmelder: O3 Tech GmbH, 27239 Twistringen (DE)
(72) Erfinder: Brals, Arend, 7963 RP RUINEN (NL); Haskamp, Clemens, 27239 Twistringen (DE)
(74) Vertreter: Eisenführ Speiser

(57) **Zusammenfassung**

Die vorliegende Erfindung betrifft ein Luftbehandlungssystem zum Reinigen von Raumluft, umfassend einen länglichen Tragkörper, insbesondere ein Rohr, mit einer vorbestimmten Tragkörperlänge, wobei der Tragkörper so ausgebildet ist, dass sich ein Kanal mit einem Kanaldurchmesser von einer Einlassseite zu einer Auslassseite erstreckt, und eine Ventilatoreinheit, die dazu eingerichtet ist, Raumluft durch den Kanal des Tragkörpers von der Einlassseite zu der Auslassseite mit einer vorbestimmten Luftförderleistung zu fördern, und eine Luftbehandlungseinheit, die dazu eingerichtet ist, Ozon in einem Ozonabschnitt innerhalb des Kanals zu erzeugen, um die durch den Kanal geförderte Raumluft in dem Ozonabschnitt mit dem erzeugten Ozon zu reinigen und die dazu eingerichtet ist, die Raumluft in einem lonisationsabschnitt innerhalb des Kanals zu ionisieren, um die durch den Kanal geförderte Raumluft in dem lonisationsabschnitt mittels Ionisation zu reinigen.

## Beschreibung

Die vorliegende Erfindung betrifft ein Luftbehandlungssystem zum Reinigen von Raumluft. Zudem betrifft die vorliegende Erfindung ein Verfahren zur Reinigung von Luft in einem geschlossenen Raum.

Luftbehandlung, die synonym auch als Luftaufbereitung verstanden werden kann, betrifft ganz generell das technische Gebiet, wie Luft bzw. Bestandteile der Luft verändert werden. Die Luftbehandlung umfasst somit nicht nur das Entziehen von Bestandteile aus der Luft, wie beispielsweise durch Filterung, sondern sie umfasst auch ein Hinzufügen von Bestandteilen zur Luft.

Das Reinigen von Luft bzw. die Luftreinigung ist ein Teilgebiet der Luftbehandlung und betrifft das technische Gebiet, gezielt ungewünschte Bestandteile, z. B. chemische oder organische Bestandteile, aus der Luft zu entfernen oder zu verringern.

Anwendungsbereiche für Luftbehandlungssysteme sind generell alle Bereiche in denen unerwünschte organische oder chemische Bestanteile in der Luft vorhanden sein können, wie beispielsweise in Tierställen, Tierzerlegeeinrichtungen, Fitnessstudios, Festzelten, Kongresshallen, Sportstätten, Schulen, Lagerhallen, Gewächshäuser, Fabriken, Tierkliniken, Hotels, Restaurants, Mensen oder in sonstigen größeren Gewerbehallen. Dort können unerwünschte und unangenehme Gerüche auftreten oder sogar Viren, Bakterien oder Pilze als Schwebstoffe in der Luft vorhanden sein, die krankheitserregend sind. Diese Belastungen können Mensch oder Tier schaden und werden deshalb auch als Schadstoffe bezeichnet.

Als ein bekannter Vorschlag zur Luftreinigung wird verwiesen auf DE 10 2007 037 440. Dieses Dokument beschreibt eine sehr aufwändige Technologie, bei der u.a. auch ein Ozonsensor gesetzt wird, dessen gemessene Werte wiederum in ein Steuer- und Regelkreis eingegeben werden, um somit die Ozonkonzentration eines Ozon (O3)-Erzeugers zu steuern.

Demnach besteht der Bedarf, unerwünschte chemische oder organische Bestandteile aller Art aus der Luft bzw. aus der Luft in einem geschlossenen Raum zu entfernen. Luft in einem geschlossenen Raum wird als Raumluft bezeichnet.

Das Entfernen oder das Verringern der unerwünschten Bestandteile der Luft kann auf unterschiedlichen Wegen erfolgen. Beispielsweise sind Systeme bekannt, die die Luft mit einem Ionisationsgerät ionisieren oder die Luft mit einem Ozongenerator mit Ozon versetzen, um unangenehme Gerüche oder organische Stoffe aus der Luft zu entfernen.

Dabei ist zudem bekannt, dass ganze Räume einmalig für einen längeren Zeitraum in eine Ozonatmosphäre versetzt werden, um organische Stoffe, wie beispielsweise Bakterien, Viren oder Pilze abzutöten. Es wird also eine Desinfektion mit Ozon durchgeführt, das auch als O₃ bekannt ist. Problematisch bei einer Luftreinigung mit Ozon ist, das Ozon in einer zu hohen Konzentration vermieden werden sollte um Einflüsse auf das gesundheitliche Wohlbefinden zu vermeiden. Ozon kann aber genutzt werden, um andere Schadstoffe zu binden und aus der Luft zu entfernen. Wir ein geschlossener Raum mit einer Ozonatmosphäre zur Luftreinigung in einer höheren Konzentration beaufschlagt, sollte dieser dementsprechend nicht ohne Schutzmaßnahmen betreten werden. Dies ist besonders für Gewerbebetriebe nachteilig, da es zu betrieblichen Stillstandzeiten und damit zu finanziellen Verlust kommt. Daneben ist eine einmalige Luftreinigung keine dauerhafte Lösung, um Räume dauerhaft belastungsarm oder -frei betreiben zu können. Nach einer einmaligen Reinigung ist die Belastung zwar kurzzeitig niedrig, nach einiger Zeit kann es aber erneut zu Verunreinigungen kommen und die Verunreinigung der Luft nach und nach größer werden, bis zu einem erneut problematischen Maß. Deshalb ist eine dauerhafte Lösung zur Luftreinigung erstrebenswert.

Da Luftbehandlungssysteme auch für Gewerbeanwendungen vorgesehen sind, in denen Räume zumeist ein großes Volumen besitzen, besteht auch ein genereller Bedarf, dass das System nachrüstbar ist, kostengünstig erworben werden kann, energiesparend arbeitet und dabei ein ausreichendes Volumen an Raumluft reinigen bzw. entkeimen kann.

Aufgabe der vorliegenden Erfindung ist es somit, zumindest eines der oben genannten Probleme zu adressieren, insbesondere soll eine Lösung vorgeschlagen werden, wie größere Mengen Raumluft kostengünstig, kontinuierlich und effizient zu reinigen sind und Schadstoffe in der Luft möglichst gering zu halten sind. Zumindest aber soll eine Alternative zu bisher bekannten Lösungen vorgeschlagen werden bzw. konzeptionell etabliert oder erarbeitet werden.

Erfindungsgemäß wird ein Luftbehandlungssystem zum Reinigen von Raumluft gemäß Anspruch 1 vorgeschlagen.

Es wird demnach ein Luftbehandlungssystem vorgeschlagen, das dazu vorgesehen ist, Luft zu reinigen, also beispielsweise unerwünschte Schadstoffe aus der Luft zu entfernen oder zumindest zu reduzieren. Raumluft bezeichnet dabei Luft, die in einem geschlossenen Raum vorhanden ist, wie beispielsweise Luft in einem Stall, einer Maschinenhalle, in einem Fitnessstudio oder in einem Hotel. Schadstoffe können organische Belastungen aller Art sein, wie beispielsweise Sporen, Pilze, Bakterien oder Viren und die als Schwebstoffe in der Luft vorhanden sind. Schadstoffe können auch chemische Belastungen sein, wie beispielsweise Ammoniak, Ameisen- oder Buttersäuremoleküle. Das Luftbehandlungssystem ist somit dazu vorgesehen, unerwünschte Bestanteile aus der Luft in einem geschlossenen Raum zu entfernen oder zumindest zu vermindern.

Das Luftbehandlungssystem umfasst dazu einen länglichen Tragkörper. Generell wird eine beliebige Form des länglichen Tragkörpers vorgeschlagen, wobei ein länglicher Tragkörper vorliegt, wenn dessen Länge wesentlich größer ist als sein Durchmesser. In einer besonders bevorzugten Ausführungsform ist der längliche Tragkörper als ein Rohr ausgebildet, beispielsweise als Rundrohr oder als Vierkantrohr. Der Tragkörper ist aus einem strukturfesten Material ausgebildet, wie beispielsweise Metall, Holz oder ähnlichen Alternativmaterial, um als Tragkörper bzw. tragende Struktur zu dienen. An den Tragkörper können elektrische oder mechanische Komponenten befestigt werden, wie beispielsweise Befestigungselemente zur Wand- oder Deckenbefestigung oder weitere Komponenten des Luftbehandlungssystems.

Der Tragkörper weist eine vorbestimmten Tragkörperlänge auf, die beispielsweise in einem Wertebereich liegt von 1m bis 20m. Die Tragkörperlänge bezeichnet demnach die Gesamtlänge des Tragkörpers.

Der Tragkörper ist dabei so ausgebildet, dass sich ein Kanal mit einem Kanaldurchmesser von einer Einlassseite zu einer Auslassseite erstreckt. Der längliche Tragkörper ist demnach hohl ausgebildet und weist einen Kanal auf, durch den die Luft strömen kann und der sich von der Einlassseite zu der Auslassseite erstreckt. Der Streckenabschnitt zwischen der Einlassseite und der Auslassseite entspricht derTragkörperlänge. Der Kanal kann auch synonym als Luftkanal bezeichnet werden. Dieser kann sich in beliebiger Form von der Einlassseite zu der Auslassseite erstrecken. Die Einlassseite ist eine Seite des Tragkörpers an der Luft in den Kanal eintritt. Die Auslassseite ist eine Seite des Tragkörpers an der Luft aus dem Kanal austritt. Der Kanaldurchmesser bezieht sich auf einen Innendurchmesser des Kanals, der beispielsweise in einem Wertebereich von 0.2m bis 2m liegt. Als ein konkretes Beispiel ist der längliche Tragkörper als ein Aluminium-Rundrohr mit einem Innendurchmesser von 0.37m und einer Länge zwischen Einlassseite und Auslassseite von 5m ausgebildet.

Das Luftbehandlungssystem umfasst zudem eine Ventilatoreinheit. Diese ist dazu eingerichtet ist, Raumluft durch den Kanal des Tragkörpers von der Einlassseite zu der Auslassseite mit einer vorbestimmten Luftförderleistung zu fördern. Als Ventilatoreinheit ist demnach eine technische Vorrichtung zu verstehen, die an oder in dem Tragkörper angeordnet ist und dazu eingerichtet ist, Raumluft durch den Kanal des Tragkörpers von der Einlassseite zu der Auslassseite zu fördern. Die Ventilatoreinheit ist also am Tragkörper angeordnet oder mindestens Lüftungstechnisch mit dieser eng verbunden, so dass die Ventilatoreinheit Raumluft durch den Kanal fördert. Die Ventilatoreinheit kann dazu beispielsweise seitlich direkt an den Tragkörper montiert sein oder mit einem Verbindungsabschnitt, wie beispielsweise mit einem Verbindungsschlauch, lüftungstechnisch mit den Tragkörper gekoppelt sein. Zudem kann die Ventilatoreinheit auch innerhalb des Tragkörpers eingeschoben sein, beispielsweise als ein eingeschobener Axialventilator. Beispiele für eine Ventilatoreinheit sind Umluftventilatoren, Gebläse, Axialventilatoren oder Lüfter. Dabei erzeugt das Luftbehandlungssystem mit der Ventilatoreinheit eine konstante Luftzirkulation, sodass die Raumluft zyklisch durch das Luftbehandlungssystem strömt und dabei gereinigt wird.

Das Luftbehandlungssystem umfasst zudem eine Luftbehandlungseinheit. Die Luftbehandlungseinheit ist eine technische Vorrichtung, die dazu eingerichtet ist, die Raumluft zu reinigen und ist dazu beispielsweise an oder in den Tragkörper fest montiert. Dabei weist die Luftbehandlungseinheit zwei Funktionen auf.

Die Luftbehandlungseinheit ist dazu eingerichtet, Ozon, dass mit der Strukturformel O₃ beschrieben wird, in einem Ozonabschnitt innerhalb des Kanals zu erzeugen, um die durch den Kanal geförderte Raumluft in dem Ozonabschnitt mit dem erzeugten Ozon zu reinigen. Dabei ist der Ozonabschnitt durch eine vorbestimmte Ozonmaximalkonzentration und eine vorbestimmte Ozonendkonzentration festgelegt. Die Ozonkonzentration des erzeugten Ozons nimmt innerhalb des Kanals entlang der Tragkörperlänge in Richtung der Auslassseite zur Ozonendkonzentration ab. Die Ozonmaximalkonzentration beschreibt einen Punkt oder Bereich innerhalb des Kanals des Tragkörpers, in dem die Konzentration des Ozons maximal ist, und beträgt beispielsweise 1.5 ppm (parts per million). Diese liegt näherungsweise in einem Bereich in dem die Luftbehandlungseinheit angeordnet ist. Die Ozonendkonzentration beschreibt einen Punkt oder einen Bereich innerhalb des Kanals des Tragkörpers, an dem eine vorbestimmte Konzentration des Ozons vorliegt, beispielsweise 0.08ppm Ozon an der Auslassseite.

Die Luftbehandlungseinheit weist demnach die erste Funktion auf, als Ozongenerator Ozon innerhalb des Kanals zu erzeugen. Das generelle Funktionsprinzip eines Ozongenerators ist dabei, dass Ozon aus Luftsauerstoff erzeugt wird, das als starkes Oxidationsmittel mit der Luft reagiert und somit chemische Abbauprozesse fördert und damit zur Geruchsbeseitigung und Desinfektion der Raumluft genutzt werden kann. Wie das Ozon in dem Rohr erzeugt wird, kann auf unterschiedliche Weise erfolgen, beispielsweise durch Bestrahlung mit einer sog. Ozonlampe oder mit einer Ozon-Einspritzung, bei der Ozon aus einem Vorratsbehälter eingespritzt wird. Bevorzugt weist die Luftbehandlungseinheit eine Ozonlampe auf, um das Ozon in dem Ozonabschnitt innerhalb des Kanals zu erzeugen. Das Ozon wird in anderen Worten vor Ort, innerhalb oder in direkter Nähe des Kanals, hergestellt und es entstehen keine schädlichen Nebenprodukte, da das Ozon in kurzer Zeit innerhalb des Kanals reagiert.

Durch die zuvor beschriebene Eigenschaft des Ozons als reaktionsfreudiges Molekül nimmt die Konzentration des Ozons entlang des Weges durch den Kanal zur Auslassseite hin ab, wenn Raumluft durch den Kanal gefördert wird. Das Ozon bindet im Grunde die unerwünschten Bestandteile aus der Raumluft, weshalb die Konzentration des Ozons in Richtung der Auslassseite abnimmt. Anhand der Ozonkonzentration kann somit ein Ozonabschnitt festgelegt werden, der ein Längenabschnitt innerhalb des Kanals des Tragkörpers beschreibt, und dessen Anfangspunkt und Endpunkt durch die Ozonmaximalkonzentration und die Ozonendkonzentration definiert sind. Beispielsweise kann als Anfangspunkt des Ozonabschnittes beispielsweise die Ozonmaximalkonzentration von 1.5ppm festgelegt werden und als ein Endpunkt die Ozonendkonzentration von 0.08ppm. Die Bestimmung der Ozonkonzentration kann durch Messung oder Berechnung erfolgen und kann sich je nach Anordnung der Luftbehandlungseinheit am oder im Tragkörper unterscheiden.

Die Luftbehandlungseinheit ist zudem dazu eingerichtet, die Raumluft in einem lonisationsabschnitt innerhalb des Kanals zu ionisieren, um die durch den Kanal geförderte Raumluft in dem Ionisationsabschnitt mittels Ionisation zu reinigen, wobei der Ionisationsabschnitt durch eine vorbestimmte erste und zweite Ionisierungsintensität festgelegt ist. Die erste und zweite Ionisierungsintensität kann beispielsweise den beiden Punkten innerhalb des Kanals entsprechen, an denen begonnen bzw. aufgehört wird, die geförderte Luft zu ionisieren.

Die Luftbehandlungseinheit weist demnach die zweite Funktion auf, als Ionisationsgerät zu arbeiten. Das generelle Funktionsprinzip eines Ionisationsgeräts ist, dass Luftpartikel ionisiert werden, die chemische Abbauprozesse fördern und somit zur Geruchsbeseitigung und Desinfektion der Raumluft genutzt werden können. Durch die Ionisation entstehen sogenannte Radikale, also reaktionsfreudige Atome oder Moleküle, die mit anderen Luftpartikeln reagieren. Wie die Luft ionisiert wird, kann auf unterschiedliche Weise erfolgen, beispielsweise durch ein lonisator mit Koronaentladung oder durch Bestrahlung mit einer UV-Lampe. Bevorzugt weist die Luftbehandlungseinheit eine UV-Lampe auf, um die Raumluft in einem Ionisationsabschnitt innerhalb des Kanals mittels UV-Bestrahlung zu ionisieren.

Demnach wird vorgeschlagen eine Ozonatmosphäre nur innerhalb des Tragkörpers bzw. in dem Kanal zu erzeugen, der bevorzugt als Rohr ausgebildet ist. Vorteilhaft ist daran, dass nicht der ganze Raum in eine schädliche Ozonatmosphäre versetzt wird. Zudem kann durch einen konstanten Betrieb der Ventilatoreinheit nach und die Umgebungsluft umgewälzt werden, sodass die Raumluft mehrfach durch das Luftbehandlungssystem zirkuliert. Damit wird kontinuierlich die Luftbelastung reduziert und auch desodoriert. Durch ständige Zirkulation wird somit die Luftqualität nach und nach verbessert.

In anderen Worten wird die Verwendung eines kombinierten Ozon- und UV-Systems zur Behandlung und Umwälzung der Luft vorgeschlagen, die zur Verringerung von Gerüchen beiträgt, beispielsweise aufgrund von Ammoniak in der Luft. Das vorgeschlagene System kann insbesondere in Ställen für Schweine, Hühner, Truthähne, Rinder und Schafe zum Tierschutz beitragen. Außerdem wird die Gesundheit das Menschen weniger stark durch Bakterien, Viren, Pilze oder Sporen belastet. Es wird also vorgeschlagen ein Ozon- und UV-C-System zu verwenden, das innerhalb des Kanals, der auch als Reaktionskammer aufgefasst werden kann, eingesetzt wird, und mit einer Ventilatoreinheit, wie ein Umluftventilator, die Raumluft durch den Kanal fördert. In dem Kanal wird dann das Ozon in Kombination mit UV-Licht erzeugt und zur Luftbehandlung genutzt.

Mit dem vorgeschlagenen System wird die Luft im gewünschten Behandlungsraum, der sich in einem Gebäude oder einem Stall befindet, mit einer vorbestimmten Frequenz umgewälzt, so dass die Luft desodoriert wird und während jedes Durchgangs desinfiziert wird. Der Geruch und das Ammoniak werden dann beispielsweise in dem Stall, in dem sich die Tiere befinden, effektiv abgebaut. Demnach wird eine Desodorierung und Desinfektion der Raumluft auf sichere Weise ermöglicht, ohne schädliche Nebenwirkungen, wie eine zu hohe Ozonbelastung im Raum.

Das vorgeschlagene Luftbehandlungssystem erreicht zudem den wichtigen Effekt, dass in dem jeweiligen geschlossenen Raum bzw. Behandlungsraum eine konstante Luftzirkulation entsteht, die den Ursachen der Luftbelastung entgegenwirkt. Diese vorgeschlagene Luftzirkulation enthält aufgrund des länglichen Tragkörper kein Ozon, da dieses innerhalb des Kanals reagiert. Demnach wird ein Luftbehandlungssystem bereitgestellt, dass nicht nur eine angenehme Luftzirkulation in geschlossenen Räumen erzeugen kann, sondern auch die Luft zyklisch nach und nach von Belastungen bzw. Schadstoffen in der Raumluft befreit.

Damit wird die Umwelt für Mensch und Tier erheblich verbessert, was auch zu weniger Infektionen der Atemwege bei den Tieren führt. Zudem wird die Ammoniakemission verringert. Die Verwendung eines solchen Luftbehandlungssystem verbessert zudem die Funktion eines bereits installierten Luftwäschers, der beispielsweise in landwirtschaftlichen Unternehmen eingesetzt wird, und wirkt sich auch auf die Verwendung von Chemikalien, Säuren und Laugen für die Umwelt positiv aus, da diese eingespart werden können.

Aufgrund des konstruktiven Aufbaus kann das System zudem in kleinen und großen Räumen eingesetzt werden, in bestehenden Gebäuden nachgerüstet oder für Neubauten eingesetzt werden. Es ermöglicht eine Installation ohne strukturelle Änderungen der Gebäude bzw. Räume. Zudem hat das System einen sehr geringen Energieverbrauch und kann ohne besondere Genehmigungen angewendet werden.

Vorzugsweise wird vorgeschlagen, dass die vorbestimmte Tragkörperlänge des Tragkörpers in Abhängigkeit einer vorbestimmten Luftförderdauer festgelegt wird, wobei die Luftförderdauer eine Zeitdauer beschreibt, die die durch den Kanal geförderte Luft benötigt, um von der Einlassseite zu der Auslassseite mittels der Ventilatoreinheit gefördert zu werden. Besonders bevorzugt wird vorgeschlagen, dass die Luftförderdauer in Abhängigkeit der Ozonendkonzentration festgelegt wird. In einer besonderes bevorzugten Ausführungsform wird die Tragkörperlänge so groß gewählt, dass die Ozonendkonzentration der geförderten Raumluft bei Austritt an der Auslassseite vorliegt.

Demnach wird vorgeschlagen, dass die Länge des Tragkörpers an die Ventilatoreinheit angepasst wird und dessen Luftförderleistung bzw. Volumenstrom berücksichtigt wird. Die Luftförderleistung wird dabei in m3/h angeben und beträgt beispielsweise 12.500m³/h. Die Förderleistung ist auch als Volumenstrom bekannt. Die Luftförderdauer beschreibt dabei eine Zeitdauer, die die Raumluft bzw. ein Partikel der Raumluft benötigt, um von der Eintrittsseite entlang der Tragkörperlänge zur Austrittsseite gefördert zu werden. Es wird somit vorgeschlagen, die Luftförderdauer so groß zu wählen, dass das erzeugte Ozon innerhalb des Rohrs vollständig reagiert bzw. die Ozonendkonzentration am Luftauslass vorliegt.

Vorzugsweise wird vorgeschlagen, dass die vorbestimmte Tragkörperlänge des Tragkörpers in Abhängigkeit des Ozonabschnittes festgelegt wird und die vorbestimmte Ozonendkonzentration unmittelbar an der Auslassseite anliegt.

Demnach wird vorgeschlagen, dass die Länge des Tragkörpers genau so lang gewählt wird, dass die vorbestimmte Ozonendkonzentration genau an der Auslassseite anliegt, also am Ende bzw. am Luftauslass des Tragkörpers. Vorteilhaft ist daran, dass die Länge des Tragkörpers maximal kurz ist aber dennoch der gewünschte Effekt erreicht wird, dass im Wesentlichen kein Ozon an der Auslassseite austritt. So kann Material für das Rohr eingespart werden und das System kompakt aufgebaut werden.

Vorzugsweise wird vorgeschlagen, dass der Kanaldurchmesser des Tragkörpers größer ist als 0.2m. In einer besonders bevorzugten Ausführungsform liegt der Kanaldurchmesser in einem Wertebereich liegt von 0.2m bis 2m, insbesondere beträgt der Kanaldurchmesser 0.37m oder 0.5m.

Zusätzlich oder alternativ wird vorgeschlagen, dass die Tragkörperlänge des Tragkörpers größer ist als 1m. In einer besonders bevorzugten Ausführungsform liegt die Tragkörperlänge in einem Wertebereich von 1m bis 20m, insbesondere beträgt die Tragkörperlänge 5m oder 7.5m.

Demnach wird ein Tragkörper vorgeschlagen, dessen konstruktive Maße für einen großen Luftdurchsatz ausgelegt sind. Damit ist das Luftbehandlungssystem besonders für große Räume geeignet ist, wie Ställe oder Hallen.

Vorzugsweise wird vorgeschlagen, das die Ozonmaximalkonzentration größer ist als 0.2ppm. In einer besonders bevorzugten Ausführungsform liegt die Ozonmaximalkonzentration in einem Wertebereich von 1ppm bis 2ppm. Zudem wird vorzugsweise vorgeschlagen, dass die Ozonkonzentration entlang der Tragkörperlänge in Richtung des Auslasses zu einer Ozonendkonzentration kleiner als 0.15ppm abnimmt. In einer besonders bevorzugten Ausführungsform nimmt die Ozonkonzentration zu einem Wert in einem Wertebereich von 0,01ppm bis 0.15ppm ab.

Die Abkürzung ppm steht für den gebräuchlichen englischen Ausdruck "parts per million" und entspricht einer gängigen Stoffmengenangabe. Im vorliegenden Fall beschreibt die Einheit demnach die Ozonkonzentration, die innerhalb des Kanals des Tragkörpers vorliegt. Grundsätzlich kann die Ozonmaximalkonzentration in ein Verhältnis zum Energieverbrauch einer Ozon erzeugenden Vorrichtung gesetzt werden. Dabei ist der Energieverbrauch höher, je höher die maximale Ozonkonzentration ist. Es wurde dabei erkannt, dass bei einer Ozonmaximalkonzentration ab 0.2ppm eine ausreichende Luftreinigung erzielt werden kann und mit einer Ozonmaximalkonzentration in einem Wertebereich von 1ppm bis 2ppm eine effiziente Luftreinigung bei gleichzeitiger energiesparender Arbeitsweise des Ozongenerators vorliegt.

Vorzugsweise wird vorgeschlagen, dass die Luftbehandlungseinheit eine Ozonlampe zur kontinuierlichen Erzeugung des Ozons aufweist, wobei die Ozonlampe dazu eingerichtet ist, Ozon mittels elektromagnetischer Strahlung in einem Wellenlängenbereich von 175nm bis 195nm zu erzeugen, insbesondere mit einer Wellenlänge von 185nm.

Demnach wird vorgeschlagen, bevorzugt eine Ozonlampe als Ozongenerator zur Erzeugung des Ozons innerhalb des Kanals des Tragkörpers zu verwenden. Eine Ozonlampe ist eine UV-Lampe, die durch eine besondere Wellenlänge bzw. einen Wellenlängenbereich im UV-Bereich charakterisiert ist, nämlich durch einen Wellenlängenbereich von 175nm bis 195nm. Die Ozonlampe weist ein Leuchtmittel zur Erzeugung von UV-Licht auf, um die Raumluft im lonisationsabschnitt innerhalb des Kanals zu ionisieren. UV ist dabei eine gängige Abkürzung für Ultraviolett, die elektromagnetische Strahlung im optischen Frequenzbereich von Licht mit kürzeren Wellenlängen als das für den Menschen sichtbare Licht beschreibt. Eine solche Ozonlampe ist zudem zur kontinuierlichen Ozonerzeugung geeignet. Der Erzeugung des Ozons liegt das Grundprinzip zugrunde, dass durch kontinuierliche Bestrahlung mit UV-Licht in der bestimmten Wellenlänge Ozon aus der Luft erzeugt wird. Zudem stellt die Ozonlampe sicher, dass der durchströmende Luftstrom permanent dem bestimmten UV-Licht ausgesetzt wird, um das Ozon bilden.

Vorzugsweise wird vorgeschlagen, dass die Luftbehandlungseinheit eine UV-Lampe zum kontinuierlichen Ionisieren der Raumluft aufweist, wobei die UV-Lampe dazu eingerichtet ist, UV-Licht mittels elektromagnetischer Strahlung in einem Wellenlängenbereich von 200nm bis 480nm zu erzeugen.

In einer besonders bevorzugten Ausführungsform ist die UV-Lampe dazu eingerichtet ist, UV-C-Licht mittels elektromagnetischer Strahlung in einem Wellenlängenbereich von 200nm bis 280nm zu erzeugen, insbesondere mit einer Wellenlänge von 254nm. Die UV-Lampe strahlt in diesem Wellenlängenbereich demnach nur UV-C-Licht aus, das durch einen Wellenlängenbereich von 100nm-280nm charakterisiert ist. In einer besonders bevorzugten Ausführungsform ist die UV-Lampe als Niederdruck-UV-Lampe ausgebildet, beispielsweise als Niederdruck-Quecksilberdampflampe.

Demnach wird vorgeschlagen, bevorzugt eine UV-Lampe als Ionisationsgerät zu verwenden, um die Raumluft in dem Ionisationsabschnitt innerhalb des Kanals zu ionisieren. Demnach wird Raumluft innerhalb des Kanals mit einer bestimmten Ultraviolettstrahlung bestrahlt, die von einer UV-Lampe ausgestrahlt wird. Die UV-Lampe weist somit ein Leuchtmittel auf. Der Methode zur Luftionisation liegt das Grundprinzip zugrunde, dass durch Bestrahlung mit Licht Schadstoffe oder -gase, wie flüchtige organische Stoffe oxidiert und damit der Luft entzogen werden.

Vorzugsweise wird vorgeschlagen, dass die Ventilatoreinheit innerhalb des länglichen Tragkörper oder lüftungstechnisch an dem länglichen Tragkörper an der Einlassseite angeordnet ist und vorzugsweise als Axialventilator ausgebildet ist. Demnach wird eine eingeschobene Ventilatoreinheit vorgeschlagen, die an einer beliebigen Stelle innerhalb des Kanals angeordnet sein. In einer besonders bevorzugten Ausführungsform ist die Ventilatoreinheit innerhalb des Kanals an der Einlassseite angeordnet. Damit wird ein robustes und kompaktes Luftbehandlungssystem bereitgestellt.

Vorzugsweise wird vorgeschlagen, dass die Ventilatoreinheit eine Luftförderleistung aufweist die größer ist als 500m³/h. In einer besonders bevorzugten Ausführungsform liegt die Luftförderleistung in einem Wertebereich von 1.000m³/h bis 20.000m³/h. Diese Förderleistung eignet sich besonders für große Räume, um beispielsweise innerhalb eines Tages mehrere Zyklen einer Luftwälzung umsetzen zu können. Mit dieser Förderleistung kann zudem in großen Räumen eine kontinuierliche Raumluftzirkulation erreicht werden. Die Luftförderleistung kann an den jeweiligen Einsatzort angepasst sein und zudem abhängig davon sein, wie häufig die Luft pro Zeit umgewälzt werden soll, beispielsweise pro Tag. In einer besonders bevorzugten Ausführungsform wird die Ventilatoreinheit von einer Antriebseinrichtung angetrieben, vorzugsweise von einem Elektromotor, beispielsweise von einem drehzahlkonstanten Elektromotor, der auf einen vorbestimmten Drehzahlwert optimiert ist. Ein solcher Motor kann besonders energieeffizient betrieben werden.

Vorzugsweise wird vorgeschlagen, dass die Ventilatoreinheit für einen Dauerbetrieb eingerichtet ist, um Raumluft zyklisch durch das Luftbehandlungssystem zu fördern. In einer besonders bevorzugten Ausführungsform ist der Dauerbetrieb ein kontinuierlicher Dauerbetrieb, also ein unterbrechungsfreier Betrieb der Ventilatoreinheit, so dass ein kontinuierlicher und stetiger Luftstrom dauerhaft generiert wird. In einer alternativen besonders bevorzugten Ausführungsform ist der Dauerbetrieb ein pulsierender Dauerbetrieb, also ein Ein- und Ausschaltbetrieb bzw. Wechselbetrieb der Ventilatoreinheit, so dass ein kontinuierlicher und stetiger Luftstrom zyklisch für eine vorbestimmte und vorgebbare Zeitdauer generiert wird.

Zusätzlich oder alternativ wird vorgeschlagen, dass die Luftbehandlungseinheit für einen Dauerbetrieb eingerichtet ist, um Raumluft zyklisch innerhalb des Kanals zu reinigen. Analog zur Ventilatoreinheit ist der Dauerbetrieb der Luftbehandlungseinheit in einer besonders bevorzugten Ausführungsform ein kontinuierlicher Dauerbetrieb, also ein unterbrechungsfreier Betrieb, so dass die den Kanal durchströmende Luft dauerhaft und stetig mit der Luftbehandlungseinheit gereinigt wird. In einer alternativen besonders bevorzugten Ausführungsform ist der Dauerbetrieb ein pulsierender Dauerbetrieb, also ein Ein- und Ausschaltbetrieb bzw. Wechselbetrieb der Luftbehandlungseinheit, so dass die den Kanal durchströmende Luft zyklisch mit der Luftbehandlungseinheit gereinigt wird.

Der Dauerbetrieb ermöglicht, dass die Raumluft dauerhaft im Raum zirkuliert und die Luft zyklisch gereinigt wird. Der kontinuierliche Dauerbetrieb ist vorteilhaft, da ein konstanter Luftstrom generiert wird bzw. eine konstante Luftreinigung erfolgt. Der pulsierende Dauerbetrieb ist energiesparender.

Vorzugsweise wird vorgeschlagen, dass der längliche Tragkörper Befestigungsmittel zur Befestigung des Luftbehandlungssystems an einer Wand und/oder an einer Decke aufweist. In einer besonders bevorzugten Ausführungsform ist das Befestigungsmittel als Aufhängung ausgebildet ist, beispielsweise als Rohraufhängung. Damit kann das gesamte Luftbehandlungssystem an einer Decke oder an einer Wand aufgehängt werden, wobei sich eine besonders effiziente Luftreinigung einstellt, wenn das Luftbehandlungssystem in einem oberen Drittel des Raumes montiert wird. Da sich Raumluft bekanntlich erwärmt und warme Luft nach oben steigt und dort verbleibt, ist warme Luft stärker belastet, als kalte Luft.

Zudem ist die an der Auslassseite austretende Raumluft durch die Luftreinigung etwas erhitzt, so dass das Luftbehandlungssystems zusätzlich als Heizung arbeiten.

Generell versteht sich, dass bei der Auslegung oder Projektierung des zuvor beschriebenen Luftbehandlungssystems beispielsweise das Raumvolumen des geschlossenen Raumes und die gewünschte Anzahl von Raumluftzyklen pro Zeit zu berücksichtigen ist. Daran wird die Luftförderleistung der Ventilatoreinheit angepasst und zudem die konstruktiven Maße des Tragkörpers festgelegt, wie den Kanaldurchmesser und die Tragkörperlänge. Zudem wird für die Bestimmung der Länge des Tragkörpers die Erzeugungsrate des Ozons der Luftbehandlungseinheit bzw. des Ozongerators berücksichtigt.

Zudem wird erfindungsgemäß ein Verfahren zur Reinigung von Luft in einem geschlossenen Raum vorgeschlagen. Das Verfahren umfasst dabei die Schritte:
- Bereitstellen eines Luftbehandlungssystems umfassend einen länglichen Tragkörper, insbesondere ein Rohr, mit einer vorbestimmten Tragkörperlänge, wobei der Tragkörper so ausgebildet ist, dass sich ein Kanal mit einem Kanaldurchmesser von einer Einlassseite zu einer Auslassseite erstreckt, eine Ventilatoreinheit, die dazu eingerichtet ist, Raumluft durch den Kanal des Tragkörpers von der Einlassseite zu der Auslassseite mit einer vorbestimmten Luftförderleistung zu fördern, und eine Luftbehandlungseinheit, die dazu eingerichtet ist, Ozon (O₃) in einem Ozonabschnitt innerhalb des Kanals zu erzeugen, um die durch den Kanal geförderte Raumluft in dem Ozonabschnitt mit dem erzeugten Ozon zu reinigen, wobei der Ozonabschnitt durch eine vorbestimmte Ozonmaximalkonzentration und eine vorbestimmte Ozonendkonzentration festgelegt ist, wobei die Ozonkonzentration des erzeugten Ozons von der Ozonmaximalkonzentration entlang der Tragkörperlänge in Richtung der Auslassseite zur Ozonendkonzentration abnimmt, und die Luftbehandlungseinheit dazu eingerichtet ist, die Raumluft in einem Ionisationsabschnitt innerhalb des Kanals zu ionisieren, um die durch den Kanal geförderte Raumluft in dem Ionisationsabschnitt mittels Ionisation zu reinigen, wobei der Ionisationsabschnitt durch eine vorbestimmte erste und zweite Ionisierungsintensität festgelegt ist,
- Montieren des Luftbehandlungssystems an einer Decke des geschlossenen Raums oder in einem oberen Bereich an einer Wand des geschlossenen Raums, um das Luftbehandlungssystems in einem klimatechnisch mit Schadgasen angehäuften Bereich zu montieren, vorzugsweise in einem oberen Drittel des geschlossenen Raumes, und
- Reinigen der Raumluft mit dem Luftbehandlungssystem.

Demnach wird vorgeschlagen, das Luftbehandlungssystems in dem geschlossenen Raum, dort wo die Luftreinigung erfolgen soll, in einem klimatechnisch mit Schadgasen bzw. Schadstoffen angehäuften Bereich zu montieren. Dieser entsteht meistens im oberen Drittel des Raumes. Deswegen ist dieser Bereich vorzugsweise zu benutzen. Es wurde erkannt, dass das oberes Drittel deshalb vorteilhaft ist, weil sich die Raumluft erwärmt, die warme Luft nach oben steigt und die warme Luft stärker belastet ist, als kalte Luft. Zudem werden in vielen Produktionsstätten Verdampfer eingesetzt, insbesondere Schlachtanlagen, die ebenfalls relativ weit oben im Raum montiert sind und die Verdampfer somit zunächst einer Reinigung unterzogen werden können.

Vorzugsweise wird vorgeschlagen, dass die Tragkörperlänge in Abhängigkeit der vorbestimmten Ozonendkonzentration an der Auslassseite festgelegt wird und zusätzlich oder alternativ in Abhängigkeit der Luftförderdauer der Ventilatoreinheit festgelegt wird.

Vorzugsweise wird vorgeschlagen, dass das Luftbehandlungssystems nach einem der Ausführungsformen ausgebildet ist. Demnach wird vorgeschlagen, das Luftbehandlungssystem wie zuvor beschrieben auszubilden.

Der Vorteil des erfindungsgemäßen Luftbehandlungssystems wie auch des erfindungsgemäßen Verfahrens zur Reinigung von Luft gegenüber bestehenden Lösungen, z.B. bekannt aus DE 10 2007 037 440 besteht darin, dass eine einfachere Lösung geschaffen wird, die mit weniger Hardware auskommt, z.B. ohne einen Ozonsensor am Ausgang des Rohrs (jedenfalls ist ein solcher Sensor nicht zwingend notwendig), weil der erfindungsgemäßen Lösung vorbestimmte Tragkörperlänge (Rohrlänge) des Tragkörpers in Abhängigkeit einer vorbestimmten Luftförderdauer festgelegt bzw. ausgelegt ist, wobei die Luftförderdauer die Zeitdauer beschreibt, die die durch den Kanal geförderte Luft benötigt, um von der Einlassseite zur Auslassseite mittels der Ventilatoreinheit gefördert zu werden und die Luftförderdauer wiederum in Abhängigkeit der Ozonendkonzentration am Ausgang festgelegt bzw. ausgelegt wird, also die Ozonendkonzentration am der Auslassseite des Tragkörpers (pro Rohr) stets eine bestimmte Quantität unterschreitet. Durch die erfindungsgemäße Lösung kommt man daher bei Bedarf auch mit weniger Hardware, z.B. ohne Ozonsensor und ohne Regelkreis wie in DE 10 2007 037 440 aus, um dennoch eine optimale Luftreinigung zu bewirken. Mit der erfindungsgemäßen Lösung werden damit strukturelle Fehler wie z.B. falsche Einstellungen des Ozonsensors, dessen Auswahl, falsche Reglungslegung, defekte Schalter etc. vermieden.

Wenn die Luftbehandlungseinheit eine UV-Lampe zum kontinuierlich Ionisieren der Raumluft aufweist, wobei die UV-Lampe dazu eingerichtet ist, UVC-Licht mittels elektromagnetischer Strahlung in einem Wellenlängenbereich von 200nm bis 280nm zu erzeugen, insbesondere mit einer Wellenlänge von 254nm kann eine über dem Stand der Technik nicht erreichte Luftreinigung erzielt werden.

Ein weiterer Vorteil der erfindungsgemäßen Systems besteht darüber hinaus auch darin, dass es nach Fertigstellung auf einfache Art und Weise in einem Raum installiert werden kann (typische Plug and Play-Lösung), ohne das weitere aufwändige Einstellarbeiten vorgenommen werden müssen.

Die erste gutachterliche Untersuchungen und Tests zeigen, dass der Einsatz der erfindungsgemäßen Lösung zu einer Reduktion von Keimträgern, Viren oder dergleichen gegenüber der unbehandelten Kontrolle von durchschnittlich 1,25 Zehnerpotenzen, das einem Reinigungsergebnis entspricht, welches bis dato noch nicht von vergleichbaren Einrichtungen erreicht wurde.

Die vorliegende Erfindung wird nun exemplarisch anhand von Ausführungsbeispielen unter Bezugnahme auf die begleitenden Figuren näher erläutert, wobei für gleiche oder ähnliche Baugruppen dieselben Bezugszeichen verwendet werden:
- Fig. 1: zeigt schematisch eine perspektivische Ansicht eines Luftbehandlungssys-tems in einer Ausführungsform.
- Fig. 2: zeigt schematisch in einer perspektivischen Ansicht zwei in einem geschlos-senen Raum angeordnete Luftbehandlungssysteme.
- Fig. 3: zeigt schematisch Kennlinienverläufe entlang einer Tragkörperlänge eines Luftbehandlungssystems in einer Ausführungsform.

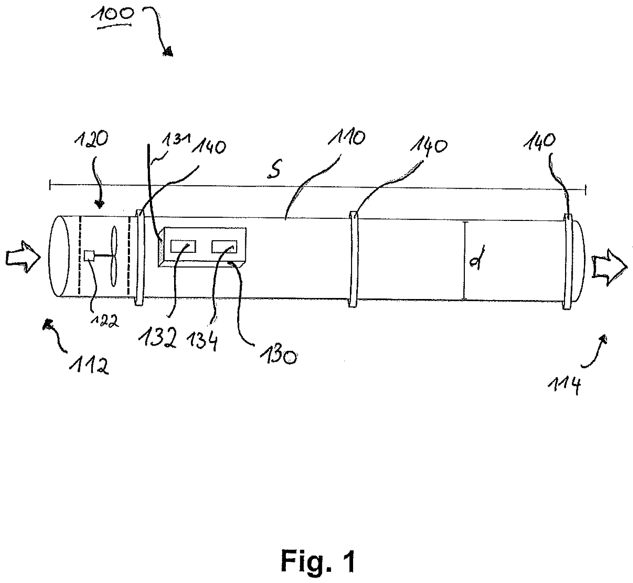

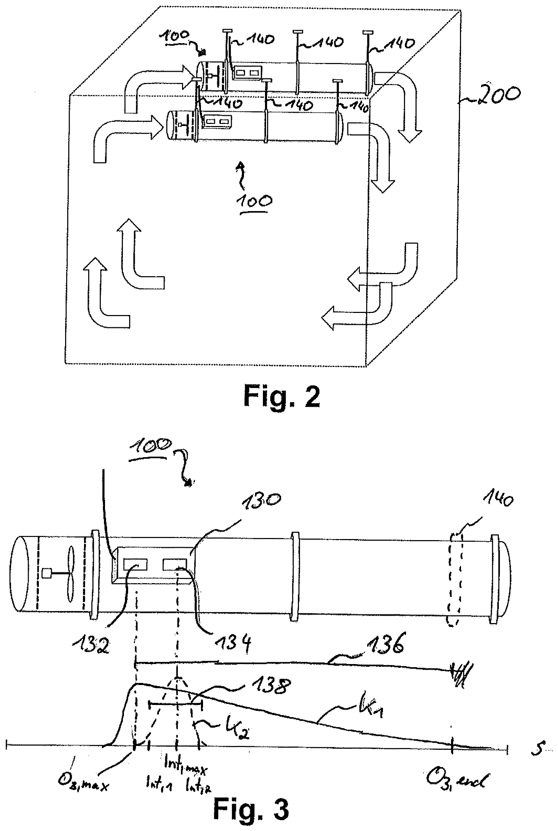

Fig. 1 zeigt schematisch eine perspektivische Ansicht eines Luftbehandlungssystems 100 zum Reinigen von Raumluft in einer Ausführungsform, mit der beispielsweise Raumluft in einem geschlossenen Raum gereinigt werden kann, wie beispielsweise der Raum 200 in der Fig. 2.

Das Luftbehandlungssystems 100 ist dazu vorgesehen Raumluft zu reinigen. Die Raumluft ist mit Pfeilen in den Figur 1 und 2 angedeutet, deren Richtung die Strömungsrichtung veranschaulichen soll. Das Luftbehandlungssystems 100 weist einen länglichen Tragkörper 110 in Form eines runden Rohres auf, der einen Kanaldurchmesser d aufweist, der auch als Innendurchmesser verstanden werden kann. Durch das Rohr 110 bzw. Rundrohr kann die Luft bzw. Raumluft hindurchströmen, nämlich von der Einlassseite 112 über die gesamte Tragkörperlänge s bis zur Auslassseite 114.

Eine Ventilatoreinheit 120 fördert die Raumluft durch den Kanal des Tragkörpers 110 von der Einlassseite 112 zu der Auslassseite 114, nämlich mit einer vorbestimmten Luftförderleistung. Die Förderleistung der Ventilatoreinheit wird dabei in Abhängigkeit der Raumgröße und der gewünschten Anzahl von Luftzirkulationen pro Zeit festgelegt. Die Ventilatoreinheit 120 ist als Axialventilator ausgebildet und innerhalb des Rohres 110 angeordnet. Die Ventilatoreinheit 120 fördert einen konstanten Luftstrom durch den Kanal, den das Rohr ausbildet. Die Ventilatoreinheit 120 ist für den Dauerbetrieb ausgelegt. Die Ventilatoreinheit 120 wird mit einer Antriebsvorrichtung 122 angetrieben, beispielsweise mit einem Elektromotor. Dieser ist für eine vorbestimmte Drehzahl für den Dauerbetrieb optimiert, um möglichst energieeffizient zu arbeiten. Über die Drehzahl und die konstruktive Ausgestaltung der Ventilatoreinheit wird die Luftförderleistung festgelegt, beispielsweise 12.500m³/h.

Eine Luftbehandlungseinheit 130 erzeugt Ozon innerhalb des Kanals, um die durch den Kanal geförderte Raumluft mit dem erzeugten Ozon zu reinigen. Die Luftbehandlungseinheit ist dabei nur schematisch von außen gezeigt und als Kasten dargestellt, der an den Tragkörper 110 festmontiert ist. Die Luftbehandlungseinheit 130 weist eine Ozonlampe 132 auf, wobei das Rechteck 132 nicht die Ozonlampe veranschaulicht, sondern den Bereich verdeutlicht, wo die Ozonlampe 132 angeordnet ist. Die Ozonlampe ist im Inneren des Rohres 110 angeordnet (nicht dargestellt) und strahlt UV-Licht in einer bestimmten Wellenlänge aus, nämlich mit 185nm. Die Luftbehandlungseinheit 130 erzeugt also mittels der Ozonlampe innerhalb des Rohres bzw. in dem Kanal Ozon. Das Ozon reagiert nach dessen Erzeugung mit der durchströmenden Raumluft, sodass Belastungen der Luft, wie organische Belastungen, gebunden werden und damit der geförderten Luft entzogen werden.

Die Luftbehandlungseinheit 130 ionisiert zudem die durch den Kanal geförderte Raumluft mittels Ionisation. Dazu weist die Luftbehandlungseinheit 130 zusätzlich zur Ozonlampe eine UV-Lampe 134 auf, wobei das Rechteck 134 nicht die UV-Lampe veranschaulicht, sondern den Bereich verdeutlicht, wo die UV-Lampe 134 angeordnet ist. Die UV-Lampe 134 ist ebenfalls im Inneren des Rohres 110 angeordnet (nicht dargestellt) und strahlt UV-C-Licht aus, allerdings mit einer anderen Wellenlänge als die Ozonlampe 132. Die UV-Lampe ist als Niederdruck-UV-Quecksilberdampf ausgebildet und bestrahlt die durchströmende Luft mit einer Wellenlänge von etwa 254nm, nämlich 253,7nm. Durch die Bestrahlung oxidieren die Belastungen in der Luft, so dass diese verringert bzw. reduziert werden.

Die Luftbehandlungseinheit 130 ist demnach als eine Art Kombigerät zu verstehen, das dazu eingerichtet ist, gleichzeitig zwei unterschiedliche Arten der Luftreinigung umzusetzen, nämlich mit der Ozonlampe 132 und mit der UV-Lampe 134. Damit kann eine besonders effiziente Luftreinigung umgesetzt werden und zudem tritt kein Ozon in den Raum ein, da dieses innerhalb des Rohres mit der geförderten Luft reagiert und an der Auslassseite gar nicht mehr oder nur noch zu einem sehr geringen maß vorhanden ist.

Zudem wird eine Stromversorgung 131 in der Fig. 1 dazu eingesetzt, das Luftbehandlungssystems 100 mit Strom zu versorgen, wie beispielsweise die Ventilatoreinheit 120 und die Luftbehandlungseinheit 130.

Das Luftbehandlungssystem 100 weist zudem drei Befestigungselemente 140 auf, mit denen das Rohr 110 an einer Decke oder in einem oberen Wandbereich aufgehängt werden kann.

Fig. 2 zeigt schematisch in einer perspektivischen Ansicht zwei in einem geschlossenen Raum 200 angeordnete Luftbehandlungssysteme 100 zum Reinigen von Raumluft, wie beispielsweise in der Figur 1 oder 3 gezeigt.

Die Luftbehandlungssysteme 100 sind dabei mit mehreren Befestigungselementen 140 an der Decke des Raumes 200 montiert, die als Rohraufhängung ausgebildet sind. Es ist somit vorgesehen, auch mehrere Luftbehandlungssysteme 100 in einem Raum zu montierten, um die Luftreinigungsrate zu erhöhen. Durch die beiden Luftbehandlungssysteme 100 entsteht eine Luftzirkulation im Raum 200, die über die Pfeile in der Fig. 2 angedeutet ist. Die Raumluft in dem Raum 200 wird entsprechend zyklisch durch die beiden Luftbehandlungssysteme 100 gefördert. Die Luftbehandlungssysteme 100 sind mit Rohraufhängungen 140 in einem Deckenbereich bzw. im oberen Drittel des Raumes 200 angeordnet. Mit dieser Anordnung und der zyklischen Zirkulation der Raumluft wird entsprechend eine dauerhafte Luftreinigung und effiziente Luftreinigung bereitgestellt, ohne das Ozon in den Raum gelangt, wie zuvor beschrieben.

Als ein konkretes Beispiel, weist der Raum 200 ein Raumvolumen von 37.500m³ auf. Die Luftförderleistung der beiden Ventilatoreinheiten beträgt 12.500m³/h. Daraus ergibt sich, dass die vollständige Raumluft alle 1.5 Stunden gereinigt wird bzw. auf den Tag gerechnet, 16x pro Tag die vollständige Raumluft gereinigt wird.

Fig. 3 zeigt schematisch ein Diagramm mit zwei schematischen Kennlinienverläufen K1 und K2 und ein Luftbehandlungssystem 100, wie beispielsweise in der Figur 1 oder 2 gezeigt.

Der Kennlinienverlauf K1 beschreibt eine Ozonkonzentration ([O₃ pro m3] bzw. ppm) in der geförderten Raumluft entlang der Tragkörperlänge s, also entlang einer Wegstrecke, innerhalb des Kanals des Luftbehandlungssystem 100. Das Luftbehandlungssystem 100 weist eine Luftbehandlungseinheit 130 mit einer Ozonlampe 132 und einer UV-Lampe 134 auf, wie beispielsweise zuvor zu den Figuren 1 und 2 beschrieben.

Wie dem Kennlinienverlauf K1 zu entnehmen ist, bildet die Ozonkonzentration innerhalb des Rohres ein Maximum aus, das als Ozonmaximalkonzentration (O_{3,max}) bezeichnet wird. Das Maximum bildet sich ungefähr dort aus, wo die Ozonlampe 132 innerhalb des Rohres 110 angeordnet ist, die das Ozon erzeugt. Strömt die Luft weiter entlang der Tragkörperlänge s nimmt die Ozonkonzentration des erzeugten Ozons innerhalb des Rohres stetig ab, da das Ozon mit der Luft reagiert und damit nach und nach abgebaut wird. Nach einiger Zeit bzw. nach einer bestimmten Wegstrecke ist die Ozonkonzentration bis zur Ozonendkonzentration O_{3,end} abgebaut. Diese kann festgelegt werden, beispielsweise als 0.08ppm. Der Längenabschnitt 136, der zwischen der Ozonmaximalkonzentration und der Ozonendkonzentration vorliegt, wird als Ozonabschnitt bezeichnet. In diesem Längenabschnitt tritt im Wesentlichen eine Reaktion des Ozons mit der geförderten Raumluft auf. Die Tragkörperlänge s des Luftbehandlungssystem 100 ist dabei wenigstens so lang, dass die Ozonendkonzentration O_{3,end} erreicht ist. Um beispielsweise Rohrmaterial einzusparen kann die Tragkörper bzw. Rohrlänge s auch mit dem Punkt zusammenfallen, an dem die Ozonendkonzentration O_{3,end} vorliegt. Dies ist mit dem gestrichelt Befestigungselement 140 in der Fig. 3 veranschaulicht. Damit ist das Rohr ausreichend lang, dass das Ozon abgebaut wird, aber maximal kurz.

Der Kennlinienverlauf K2 beschreibt eine Ionisierungsintensität mit der die geförderte Raumluft entlang der Tragkörperlänge s innerhalb des Kanals des Luftbehandlungssystem 100 ionisiert wird. Diese kann auch als Bestrahlungsrate oder Bestrahlungsstärke aufgefasst werden.

Wie dem Kennlinienverlauf K2 zu entnehmen ist, bildet die Ionisierungsintensität innerhalb des Rohres ein Maximum aus, das als maximale Bestrahlungsintensität Int_{,max} bezeichnet wird. Die maximale Bestrahlungsintensität bildet sich ungefähr dort aus, wo die UV-Lampe 132 innerhalb des Rohres 110 angeordnet ist. Der Längenabschnitt 138 der zwischen der einer ersten Ionisierungsintensität Int,1 und einer zweiten Ionisierungsintensität Int,2 liegt, wird als Ionisationsabschnitt bezeichnet, da in diesem Abschnitt im Wesentlichen die Luft ionisiert bzw. bestrahlt wird. Strömt die geförderte Raumluft entlang der Tragkörperlänge s durch den lonisationsabschnitt, werden die Belastungen in der Luft durch die Bestrahlung oxidiert, sodass diese reduziert werden. Die beiden Punkte Int,1 und Int,2 sind demnach die Punkte, an denen die Bestrahlung mit UV-Licht innerhalb des Kanals beginnt und endet.

### Bezuqszeichenliste

- 100: Luftbehandlungssystem
- 110: Tragkörper
- 112: Einlassseite
- 114: Auslassseite
- 120: Ventilatoreinheit
- 122: Antriebsvorrichtung
- 130: Luftbehandlungseinheit
- 131: Stromversorgung
- 132: Ozonlampe
- 134: UV-lampe
- 136: Ozonabschnitt
- 138: lonisationsabschnitt
- 140: Befestigungselement
- d: Kanaldurchmesser
- s: Tragkörperlänge
- O_{3,max}: Ozonmaximalkonzentration
- O_{3,end}: Ozonendkonzentration

## Patentansprüche

1. Luftbehandlungssystem (100) zum Reinigen von Raumluft umfassend
- einen länglichen Tragkörper (110), nämlich ein Rohr, mit einer vorbestimmten Tragkörperlänge (s), wobei der Tragkörper so ausgebildet ist, dass sich ein Kanal mit einem Kanaldurchmesser (d) von einer Einlassseite (112) zu einer Auslassseite (112) erstreckt,
- eine Ventilatoreinheit (120), die dazu eingerichtet ist, Raumluft durch den Kanal des Tragkörpers von der Einlassseite zu der Auslassseite mit einer vorbestimmten Luftförderleistung zu fördern, und
- eine Luftbehandlungseinheit (130), die dazu eingerichtet ist, Ozon (O₃) in einem Ozonabschnitt (136) innerhalb des Kanals zu erzeugen, um die durch den Kanal geförderte Raumluft in dem Ozonabschnitt mit dem erzeugten Ozon zu reinigen, wobei der Ozonabschnitt durch eine vorbestimmte Ozonmaximalkonzentration (O_{3,max}) und eine vorbestimmte Ozonendkonzentration (O_{3,end}) festgelegt ist, wobei die Ozonkonzentration des erzeugten Ozons von der Ozonmaximalkonzentration (O_{3,max}) entlang der Tragkörperlänge in Richtung der Auslassseite zu der Ozonendkonzentration (O_{3,end}) abnimmt, und
- die Luftbehandlungseinheit (130) dazu eingerichtet ist, die Raumluft in einem lonisationsabschnitt (138) innerhalb des Kanals zu ionisieren, um die durch den Kanal geförderte Raumluft in dem Ionisationsabschnitt mittels Ionisation zu reinigen, wobei der Ionisationsabschnitt durch eine vorbestimmte erste und zweite Ionisierungsintensität festgelegt ist und
- die vorbestimmte Tragkörperlänge des Tragkörpers in Abhängigkeit einer vorbestimmten Luftförderdauer so festgelegt und/oder ausgelegt ist, wobei die Luftförderdauer eine Zeitdauer beschreibt, die die durch den Kanal geförderte Luft benötigt, um von der Einlassseite des Tragkörpers zu der Auslassseite des Tragkörpers mittels der Ventilatoreinheit gefördert zu werden und die Luftförderdauer in Abhängigkeit der Ozonendkonzentration festgelegt und/oder ausgelegt wird und die vorbestimmte Ozonendkonzentration (O_{3,end}) einen vorbestimmten Wert nicht überschreitet, und/oder
- die Luftbehandlungseinheit (130) eine UV-Lampe (134) zum kontinuierlichen Ionisieren der Raumluft aufweist, wobei die UV-Lampe dazu eingerichtet ist, UVC-Licht mittels elektromagnetischer Strahlung in einen Wellenlängenbereich von 200nm bis 280nm zu erzeugen, insbesondere in einer Wellenlänge von 254nm.

2. Luftbehandlungssystem nach Anspruch 1, **dadurch gekennzeichnet, dass**
die vorbestimmte Tragkörperlänge (s) des Tragkörpers (110) in Abhängigkeit des Ozonabschnittes festgelegt wird und die vorbestimmte Ozonendkonzentration (O_{3,end}) unmittelbar an der Auslassseite (114) anliegt.

3. Luftbehandlungssystem nach einem der vorstehenden Ansprüchen, **dadurch gekennzeichnet, dass**
der Kanaldurchmesser (d) des Tragkörpers größer ist als 0.2m, vorzugsweise in einem Wertebereich liegt von 0.2m bis 2m, insbesondere 0.37m oder 0.5m beträgt und/oder
die Tragkörperlänge (s) des Tragkörpers größer ist als 1m, vorzugsweise in einem Wertebereich liegt von 1m bis 20m, insbesondere 5m oder 7.5m beträgt.

4. Luftbehandlungssystem nach einem der vorstehenden Ansprüchen, **dadurch gekennzeichnet, dass**
die Ozonmaximalkonzentration (O_{3,max}) größer ist als 0.2ppm, vorzugsweise in einem Wertebereich liegt von 1ppm bis 2ppm, und die Ozonkonzentration entlang der Tragkörperlänge in Richtung des Auslasses zu einer Ozonendkonzentration (O_{3,end}) kleiner als 0.15ppm abnimmt, vorzugsweise zu einer Ozonendkonzentration in einem Wertebereich von 0,01ppm bis 0.15ppm abnimmt.

5. Luftbehandlungssystem nach einem der vorstehenden Ansprüchen, **dadurch gekennzeichnet, dass**
die Luftbehandlungseinheit (130) eine Ozonlampe (132) zur kontinuierlichen Erzeugung des Ozons aufweist, wobei die Ozonlampe dazu eingerichtet ist, Ozon mittels elektromagnetischer Strahlung in einem Wellenlängenbereich von 175nm bis 195nm zu erzeugen, insbesondere mit einer Wellenlänge von 185nm.

6. Luftbehandlungssystem nach einem der vorstehenden Ansprüchen, **dadurch gekennzeichnet, dass**
die Ventilatoreinheit (120) innerhalb des länglichen Tragkörper (110) oder an dem länglichen Tragkörper (110) an der Einlassseite angeordnet ist und vorzugsweise als Axialventilator ausgebildet ist.

7. Luftbehandlungssystem nach einem der vorstehenden Ansprüchen, **dadurch gekennzeichnet, dass**
die Ventilatoreinheit (120) eine Luftförderleistung aufweist die größer ist als 500m³/h, vorzugsweise in einem Wertebereich liegt von 1.000m³/h bis 20.000m³/h.

8. Luftbehandlungssystem nach einem der vorstehenden Ansprüchen, **dadurch gekennzeichnet, dass**
die Ventilatoreinheit (120) für einen Dauerbetrieb eingerichtet ist, insbesondere für einen kontinuierlichen oder pulsierenden Dauerbetrieb eingerichtet ist und/oder
die Luftbehandlungseinheit (130) für einen Dauerbetrieb eingerichtet ist, insbesondere für einen kontinuierlichen oder pulsierenden Dauerbetrieb eingerichtet ist, um Raumluft zyklisch durch das Luftbehandlungssystem zu fördern.

9. Luftbehandlungssystem nach einem der vorstehenden Ansprüchen, **dadurch gekennzeichnet, dass**
das der Tragkörper (110) Befestigungsmittel (140) zur Befestigung des Luftbehandlungssystems an einer Wand und/oder an einer Decke aufweist, wobei das Befestigungsmittel vorzugsweise als Aufhängung ausgebildet ist, insbesondere als Rohraufhängung.

10. Verfahren zur Reinigung von Luft in einem geschlossenen Raum, umfassend die Schritte:
- Bereitstellen eines Luftbehandlungssystems (100) umfassend einen länglichen Tragkörper (110), nämlich ein Rohr, mit einer vorbestimmten Tragkörperlänge, wobei der Tragkörper so ausgebildet ist, dass sich ein Kanal mit einem Kanaldurchmesser von einer Einlassseite zu einer Auslassseite erstreckt, eine Ventilatoreinheit (120), die dazu eingerichtet ist, Raumluft durch den Kanal des Tragkörpers von der Einlassseite zu der Auslassseite mit einer vorbestimmten Luftförderleistung zu fördern, und eine Luftbehandlungseinheit (130), die dazu eingerichtet ist, Ozon (O₃) in einem Ozonabschnitt innerhalb des Kanals zu erzeugen, um die durch den Kanal geförderte Raumluft in dem Ozonabschnitt mit dem erzeugten Ozon zu reinigen, wobei der Ozonabschnitt durch eine vorbestimmte Ozonmaximalkonzentration (O_{3,max}) und eine vorbestimmte Ozonendkonzentration (O_{3,end}) festgelegt ist, wobei die Ozonkonzentration des erzeugten Ozons von der Ozonmaximalkonzentration entlang der Tragkörperlänge in Richtung der Auslassseite zur Ozonendkonzentration abnimmt, und die Luftbehandlungseinheit dazu eingerichtet ist, die Raumluft in einem Ionisationsabschnitt innerhalb des Kanals zu ionisieren, um die durch den Kanal geförderte Raumluft in dem Ionisationsabschnitt mittels Ionisation zu reinigen, wobei der Ionisationsabschnitt durch eine vorbestimmte erste und zweite Ionisierungsintensität festgelegt ist,
- Montieren des Luftbehandlungssystems an einer Decke des geschlossenen Raums oder in einem oberen Bereich an einer Wand des geschlossenen Raums, um das Luftbehandlungssystems in einem klimatechnisch mit Schadgasen bzw. Schadstoffen angehäuften Bereich zu montieren, vorzugsweise in einem oberen Drittel des geschlossenen Raumes, und
- Reinigen der Raumluft mit dem Luftbehandlungssystem.

11. Verfahren nach Anspruch 10, **dadurch gekennzeichnet, dass**
die Tragkörperlänge in Abhängigkeit der vorbestimmten Ozonendkonzentration an der Auslassseite festgelegt wird und/oder
in Abhängigkeit einer Luftförderdauer der Ventilatoreinheit festgelegt wird.

12. Verfahren nach Anspruch 10 oder 11 **dadurch gekennzeichnet, dass**,
das Luftbehandlungssystems nach einem der Ansprüche 1 bis 9 ausgebildet ist.
